(19) Europäisches Patentamt / European Patent Office / Office européen des brevets

(11) **EP 4 352 339 B1**

(12) **EUROPEAN PATENT SPECIFICATION**

(45) Date of publication and mention
of the grant of the patent:
**04.09.2024 Bulletin 2024/36**

(21) Application number: **22732261.7**

(22) Date of filing: **10.06.2022**

(51) International Patent Classification (IPC):
$F01K\ 13/02$ (2006.01)     $F22B\ 35/18$ (2006.01)
$F23C\ 1/12$ (2006.01)     $F23D\ 1/00$ (2006.01)
$F23G\ 5/033$ (2006.01)     $F23G\ 7/10$ (2006.01)
$G01K\ 17/00$ (2006.01)     $G06N\ 3/08$ (2023.01)
$G01N\ 33/22$ (2006.01)

(52) Cooperative Patent Classification (CPC):
**F22B 35/18; F01K 13/02; F23C 1/12; F23D 1/00;
F23G 5/033; F23G 7/10; F23G 7/105; G01K 17/00;**
F23G 2209/26; F23N 2221/10; G01N 33/222;
G06N 3/08

(86) International application number:
**PCT/EP2022/065842**

(87) International publication number:
**WO 2022/258811 (15.12.2022 Gazette 2022/50)**

(54) **A METHOD AND A SYSTEM FOR MONITORING AND ON-LINE DETERMINING OF A CALORIFIC VALUE OF SOLID FUEL THAT IS CURRENTLY COMBUSTED IN A BOILER**

VERFAHREN UND SYSTEM ZUR ON-LINE-ÜBERWACHUNG UND -BESTIMMUNG DES HEIZWERTES EINES FESTBRENNSTOFFES, DER DERZEIT IN EINEM KESSEL VERBRENNT WIRD

PROCÉDÉ ET SYSTÈME DE SURVEILLANCE ET DE DÉTERMINATION EN LIGNE DU POUVOIR CALORIFIQUE DU COMBUSTIBLE SOLIDE ACTUELLEMENT BRÛLÉ DANS UNE CHAUDIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2021 EP 21461552**

(43) Date of publication of application:
**17.04.2024 Bulletin 2024/16**

(73) Proprietor: **Boneffice System Sp. z o.o.
02-390 Warszawa (PL)**

(72) Inventors:
• **LASEK, Dawid**
**27-200 Starachowice (PL)**
• **KUREK, Damian**
**03-194 Warszawa (PL)**
• **ROSZKOWSKI, Jaroslaw**
**05-502 Piaseczno (PL)**
• **URBANIAK, Anita**
**11-612 Lipowo (PL)**

(74) Representative: **Kancelaria Eupatent.pl Sp. z.o.o
Ul. Kilinskiego 185
90-348 Lodz (PL)**

(56) References cited:
**EP-A1- 2 437 206     US-A- 4 969 408**

• **KUREK DAMIAN ET AL: "Systemy wsparcia decyzji operatora bloku. Zastosowanie metod deterministycznych i nowoczesnych algorytmów AI", NOWA ENERGIA, NR 5-6 (70) / 2019, 12 November 2019 (2019-11-12), Racibórz (Poland), pages 19 - 21, XP055866273, Retrieved from the Internet
<URL:https://nowa-energia.com.pl/media/magazyny_archiwalne/2019/magazyn_nowa_energia_2019_05_06.pdf> [retrieved on 20211126]**

## Description

### TECHNICAL FIELD

The present invention relates to monitoring combustion of solid fuel in a boiler and to on-line determining of a calorific value of the solid fuel currently combusted in the boiler, in particular in thermal power plants.

### BACKGROUND

Thermal power plants produce electricity as a result of energy conversions. The main component of a thermal power plant is a steam boiler, wherein fuel is combusted (usually fossil fuel - coal or natural gas, biomass, biogas or waste). The thermal energy from the steam boiler is used to heat, evaporate and superheat steam that feeds the turbine. The steam-powered turbine generates mechanical energy to drive a shaft of an electric generator. Large power plants may be equipped with several power blocks, wherein energy production processes are carried out such that they are at least partially separate (or usually independent of each other). These processes are carried out usually by means of independent elements, such as boilers, turbines, condensers, pumps, exchangers, generators and the like.

Typically, thermal boilers have an efficiency in the range of 70 to 94%. The efficiency of the boiler results from the applied solutions, wear and tear of devices, irreversibility of thermal processes and process losses resulting from imperfect control and process errors. These losses get worse along with the lifetime of the boiler, which can be up to several decades. Thus, various types of monitoring systems are used in thermal power plants. The monitoring systems are used to monitor the operation of the power plant, including the boiler, and to generate information on how to optimise the operation of the power plant.

There are known Distributed Control Systems (DCS) that collect engineering and technical information from individual points in the power plant in order to automate and control the operation of the blocks. Separate systems, such as boiler management systems (often based on neural networks) are typically used to automate the boiler operation.

Typically, DCS-based automation systems are used with implemented design process characteristics of boiler operation, obtained at the time of commissioning of the power plant or partially corrected after major overhauls. These characteristics become less and less useful as the service life and associated operational and technical changes progress. In addition, there is a lack of support for control systems with key real-time information on the calorific values of the fuel that is currently combusted in the boiler, as well as on the content of combustible parts in ash and slag. Information about the LHV (Lower Heating Value) of the fuel is necessary to accurately optimise the operation of the boiler, but in typical systems, fuel quality analysis is carried out with a significant delay, making it impossible to use the results of this analysis in real time. Possible solutions require an additional investment to upgrade the boiler infrastructure. In the case of boilers which have been in operation for several decades, solutions which do not interfere with the plant's infrastructure are preferred over new investments.

Therefore, there is a need to develop a system that monitors and determines, on-line, the calorific value of a solid fuel currently combusted in a boiler, such as to achieve these objectives without interfering with the infrastructure of the facility.

Such a system is disclosed, but only in very general terms, in Kurek Damian et al.: "Systemy wsparcia decyzji operatora bloku. Zastosowanie metod deterministycznych i nowoczesnych algorytmów AI", Nowa Energia, nr. 5-6 (70) / 2019, 12 November 2019, pages 19-21, Racib6rz (Poland).

### SUMMARY OF THE INVENTION

Information about the current calorific value of the fuel is critical to determining the amount of energy input to the boiler. Without this information, it is not possible to optimise the combustion process on-line. For example, for a coal-fired power plant, fuel quality varies by around 20% (even if the coal comes from the same mine). Therefore, it is important to know the calorific value of the fuel that is currently combusted.

The ability to achieve the required power in the block depends on the chemical energy reserves of fuel available at the solid fuel mills. Currently, the block operator has no knowledge of the energy potential available at the mills that prepare the solid fuel fed to the boiler. Information on the current calorific value would allow the determination of energy reserves both on the currently operating mill units, as well as on units that are currently shut down but ready to be switched on in case of the need to reach high loads of the power block.

Moreover, in typical known systems, the operator has no information about the quality of the fuel that is currently combusted, nor about the current efficiency of energy production. This leads to a limited ability to respond to adverse conditions by not being able to detect those adverse conditions. This also results in the possibility of suboptimal control decisions due to a lack of knowledge of the exact amount of energy input to the boiler.

The systems offered on the market require hardware investment and interference with the technological set-up of the power plant.

The system according to the invention solves the problems described above. The solution according to the invention enables precise on-line management and optimisation of the boiler operation, as well as significantly increases the operational awareness of boiler operators.

The object of the invention is a method for on-line monitoring and determining a calorific value of a solid fuel that is currently combusted in a boiler that comprises a combustion chamber with a steam production system, to which the solid fuel is fed from at least one mill connected to a hopper. The method comprises measuring, on-line during the operation of the boiler, operational data of the boiler, and operational data of at least one mill. The operational data of the boiler are measured by means of sensors and comprise at least one of: temperature, pressure, steam flow; wherein the operational data of the at least one mill are measured by means of sensors and comprise at least one of: mill power, air pressure upstream of the mill, solid fuel feeder revolutions, air temperature upstream of the mill, temperature of the dust-air mixture downstream of the mill. The method further comprises collecting historical data of measurements of the calorific value of the solid fuel and the operational data of a power block, wherein the operational data of the power block include at least one of: lower heating value, mill characteristics, fuel flow rate and boiler efficiency, operational parameters of turbine, including flows, pressures and temperatures for live steam, superheated steam and secondary steam, operational data of the boiler, operational data of at least one mill, composition of exhaust gases, power of the block. The method also includes calculating energy balances of the steam production system, based on the collected historical data and depending on the amount of thermal energy input to the turbine generator set divided by the boiler efficiency. Further, the method includes iteratively determining an efficiency of the boiler based on the historical data by: determining sets of mill characteristics depending on a calorific value of the solid fuel and operational data of the mill while combusting that solid fuel, wherein the mill characteristics include at least one operational parameter selected from the group consisting of: mill power, air pressure upstream of the mill, solid fuel feeder revolutions, air temperature upstream of the mill, temperature of the dust-air mixture downstream of the mill; determining a fuel mass flux based on the set of mill characteristics; and determining the actual calorific value of the fuel for the historical data. Next, the method includes training a model based on artificial intelligence algorithms to predict the calorific value using the historical data of fuel calorific value and measured operational data of the boiler; and determining in real time, using the trained model, the calorific value of the solid fuel that is currently combusted.

Preferably, the historical data of the calorific value of the solid fuel are determined by means of laboratory measurements carried out cyclically or by measurement systems operating continuously, for the solid fuel prior to putting the solid fuel to the hopper.

Preferably, the method further comprises measuring, on-line during the operation of the boiler, data of ambient conditions, wherein the data of ambient conditions are measured by means of a sensor and comprise at least one of: ambient air temperature, ambient air pressure, ambient air humidity.

The determined calorific value for the fuel that is currently combusted is stored in a database or the determined calorific value is directly used in other computational, analysis or advisory modules in order to optimise the boiler operation. If a plurality of mills are used, the available energy potential at the individual mills can be determined in the manner described herein.

In particular, the energy balances take into account the amount of energy received by the live steam and the secondary steam from the boiler (for the calculated secondary steam flow).

The boiler efficiency is determined by an indirect method (based on boiler losses) or by a direct method (known mass of the fuel entering the boiler).

The mill characteristics are built using current data for established historical periods prior to their use in the calculations. The mill characteristics are selected so as to minimise the error in the amount of energy supplied to the boiler.

Based on the determined energy balances, the boiler efficiency and the mill characteristics, the calorific value of the fuel is determined at particular time intervals.

The invention also relates to a system for on-line monitoring and determining a calorific value of a solid fuel that is currently combusted in a boiler that comprises a combustion chamber with a steam production system to which the solid fuel is fed from at least one mill connected to a hopper. The system comprises an interface to sensors (15-1, 15-2, 15-3, 15-4, 13-1, 17-1) for measuring operational data of the boiler and operational data of at least one mill. The operational data of the boiler comprise at least one of: temperature, pressure, steam flow. The operational data of the at least one mill comprise at least one of: mill power, air pressure upstream of the mill, solid fuel feeder revolutions, air temperature upstream of the mill, temperature of the dust-air mixture downstream of the mill. The system also comprises an archive module for collecting historical data of solid fuel calorific value measurements and operational data of the power block, wherein the operational data of the power block include at least one of: lower heating value, mill characteristics, fuel flow rate and boiler efficiency, operational parameters of turbine, including flows, pressures and temperatures for live steam, superheated steam and secondary steam, operational data of the boiler, operational data of at least one mill, composition of exhaust gases, power of the block. The system further comprises a module for calculating energy balances of the steam production system on the basis of the historical data collected in the archive module and depending on the amount of thermal energy input to the turbine generator set divided by the boiler efficiency.

The system also comprises a module for iterative determination (207-212) of boiler efficiency on the basis of historical data, configured to: determine sets of mill characteristics depending on a calorific value of the solid fuel and operational data of the mill while combusting that solid fuel, wherein the mill characteristics include at least one operational parameter selected from the group consisting of: mill power, air pressure upstream of the mill, solid fuel feeder revolutions, air temperature upstream of the mill, temperature of the dust-air mixture downstream of the mill; determine the fuel mass flux on the basis of the set of mill characteristics; and determine the actual calorific value of the fuel for historical data. The system also comprises a model based on artificial intelligence algorithms for predicting calorific value, that is trained using historical data of fuel calorific value and measured operational data of the boiler, and once trained, configured to determine in real time the calorific value of the solid fuel that is currently combusted on the basis of the currently measured operational data of the boiler.

In certain specific embodiments, the solution according to the invention provides the possibility to accurately optimise the combustion process by knowledge of the amount of energy input to the system, by implementing even more precise automatic control systems or operational decision support systems.

Moreover, in certain specific embodiments, the solution according to the invention provides the possibility of assessing the energy potential of the solid fuel mills and thus obtaining information on the ability of the power block to achieve the planned power at the currently operating mill units.

Moreover, in certain specific embodiments, the solution according to the invention provides the possibility to accurately assess on-line (i. e. on an ongoing basis) the quality of the operation of the power generation process, by determining on-line the efficiency of the boiler, and thus provides the possibility for the operator to eliminate areas that generate losses. This leads to maximising the use of the existing technical potential of the power plant.

Moreover, in certain specific embodiments, the solution according to the invention provides information to professionals responsible for operating the power block about the current calorific value of the fuel that is combusted and its susceptibility to milling, which results in increased operational awareness and enables implementation of operating practices precisely configured to the current conditions.

Moreover, in certain specific embodiments, the solution according to the invention does not require use of additional metering and hardware investments.

## BRIEF DESCRIPTION OF DRAWINGS

The object of the invention is shown by means of an embodiment in the drawing, wherein:

Fig. 1 shows a simplified diagram of the boiler;
Fig. 2 shows a flowchart of the method according to the invention;
Fig. 3 shows an example set of mill characteristics;
Fig. 4 shows a simplified diagram of the module for on-line determination of calorific value;
Fig. 5 shows a simplified flowchart of the method for determining the calorific value.

## DETAILED DESCRIPTION OF EMBODIMENT

The embodiment shown herein will be discussed based on an example of a boiler supplied with a solid fuel that is coal, but the method can be used in an equivalent manner for any other solid fuel, such as biomass.

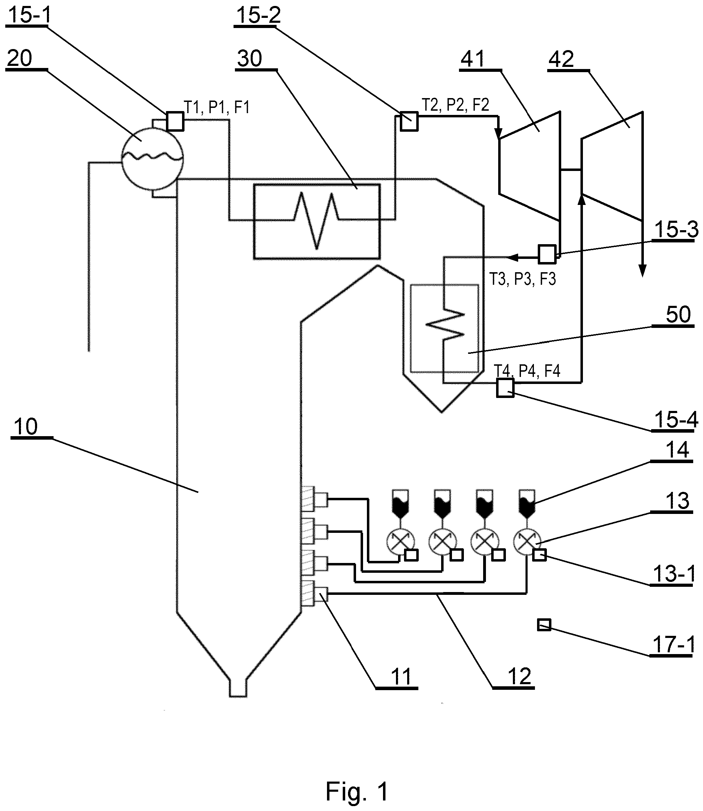

Fig. 1 shows a simplified diagram of the boiler. This diagram is based on known constructions, provided for exemplary purposes only, to illustrate an example of the system for which the method according to the invention can be used. Therefore, it is possible to apply the method also for other boilers having a similar configuration. The boiler comprises a combustion chamber 10 with a steam production system. The combustion chamber 10 comprises burners 11 to which solid fuel from mills 13 is supplied via dust-air ducts 12. Each mill 13 has its own mill hopper 14 with a solid fuel feeder that feeds the fuel to the mill. The mills 13 mill the solid fuel and separate undesirable components. Operational parameters of the mills 13 are measured by sensors 13-1 and comprise at least one of: mill power, air pressure upstream of the mill, solid fuel feeder revolutions, air temperature upstream of the mill, temperature of the dust-air mixture downstream of the mill.

Furthermore, ambient conditions are measured by at least one sensor 17-1 (i.e. a single sensor located at a designated point within the facility or a plurality of sensors located at specific locations) and comprise at least one of: ambient air temperature, ambient air pressure, ambient air humidity, i.e. data on atmospheric conditions.

A boiler drum 20 separates water from steam. Live steam having temperature T1, pressure P1 and flow F1 (measured, for example, as flow rate) is directed to a live steam superheater 30, wherein additional energy is supplied to the steam by raising its temperature and thus superheated steam having temperature T2, pressure P2 and flow F2 is fed to a highpressure section 41 of a turbine, wherein it is subject to expansion, releases some of its energy and returns to the boiler as secondary steam having temperature T3, pressure P3 and flow F3. A secondary steam superheater 40 heats the steam and the superheated steam having temperature T4, pressure P4 and flow F4 is directed to a medium-pressure and then to low-pressure section 42 of the turbine, wherein it releases its energy. The values of temperature, pressure and flow of the steam are measured by means of corresponding sensors 15-1, 15-2, 15-3, 15-4.

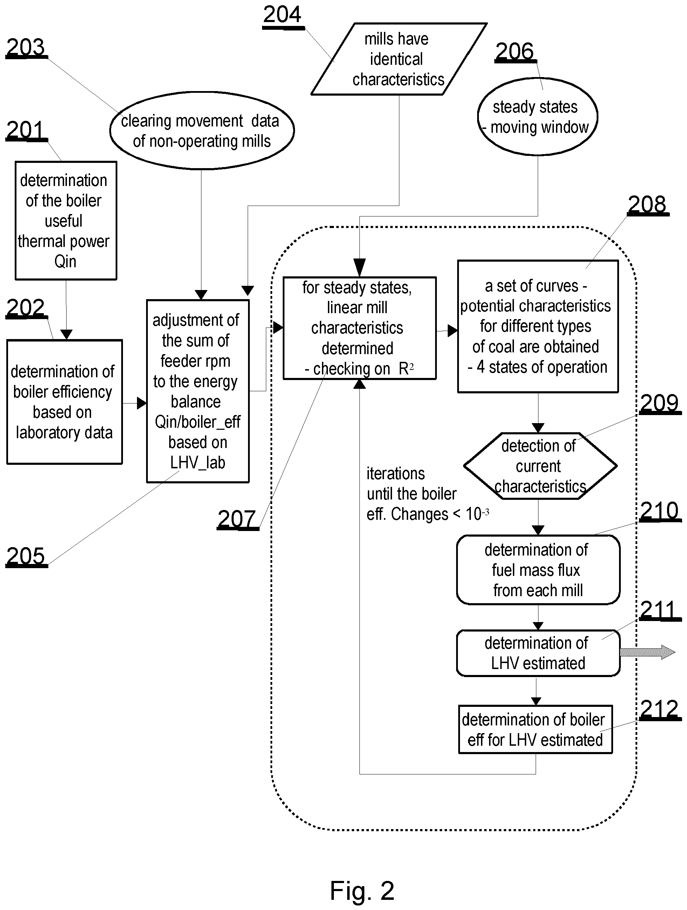

Fig. 2 shows a flowchart for carrying out the method according to the invention.

In step 201, the boiler useful thermal power (Qin) is determined, which indicates the amount of energy transferred from the boiler 10 to the turbine 41, 42 in the form of steam. At this step, the calorific value of the fuel and the fuel mass flow rate are not known yet, therefore it is not possible to calculate the efficiency from the energy output of the boiler. The boiler useful thermal power (Qin) is determined based on the data on the flow of live steam, secondary steam, the parameters of this steam and other parameters, for example using the formula:

$$Q_{in} = [(\dot{m}_{psw}-\dot{m}_{wtrysk\_psw})*(h_{psw}-h_{wz})+\dot{m}_{wtrysk\_psw}*(h_{psw}-h_{wtrysk\_psw}) + (\dot{m}_{pwt}-\dot{m}_{wtrysk\_pwt})*(h_{pwt}-h_{psw\_wylot})+\dot{m}_{wtrysk\_pwt}*(h_{pwt}-h_{wtrysk\_pwt})]/3600-Q_z$$

wherein:

$\dot{m}_{psw}$ is the flow of live steam (F2) entering the turbine (41)
$\dot{m}_{wtrysk\_psw}$ is the flow of water injected into live steam
$h_{psw}$ is the enthalpy of live steam entering the turbine
$h_{wz}$ is the enthalpy of water fed to the boiler
$h_{psw\_wylot}$ is the enthalpy of live steam leaving the turbine
$h_{wtrysk\_psw}$ is the enthalpy of water injected into live steam
$\dot{m}_{pwt}$ is the flow of secondary steam (F4) entering the turbine (42)
$\dot{m}_{wtrysk\_pwt}$ is the flow of water injected into secondary steam
$h_{pwt}$ is the enthalpy of secondary steam entering the turbine
$h_{wtrysk\_pwt}$ is the enthalpy of water injected into secondary steam
$Q_z$ is the amount of heat dissipated outside the system

In step 202, the initial efficiency of the boiler is determined on the basis of historical 24-hour - average laboratory data of calorific value and the composition of the fuel, according to the PN-EN 12952-15:2004 standard.

In step 203, processing of measurement data from the environment of the coal mills is carried out so as to remove erroneous indication values generated by currently deactivated mills 13, e.g. the error of negative revolutions of feeders of a non-operating mill 13.

In step 204, it is initially assumed that mill characteristics are identical for all of the mills 13, which corresponds to the assumption that each mill hopper 14 contains a fuel with the same properties.

Based on the data from steps 202, 203, 204, in step 205 the fuel mass flow rate is adjusted by selecting appropriate mill characteristics. The resulting fuel mass flux is related to the 24-hour-average laboratory calorific value, so that the balance of the amount of chemical energy of the fuel fed to the boiler is fulfilled. The balance relates to the amount of thermal energy input to the turbine generator set (Qin), divided by the boiler efficiency, according to the formula:

$$qPal = f\left(\sum obrMW\right) * LHV_{lab} = \frac{Q_{in}}{\eta_{boiler}}$$

wherein:

$\Sigma$ obr MW is the measured sum of the feeders revolutions
$f(\Sigma\ obrMW)$ is the fuel mass flow rate as a function of the sum of revolutions of the feeders of particular mills, and the calculations are carried out using the determined mill characteristics
$LHV_{lab}$ is the 24-hour-average laboratory calorific value
Qin is the amount of thermal energy input to the turbine generator set
$\eta_{boiler}$ is the boiler efficiency

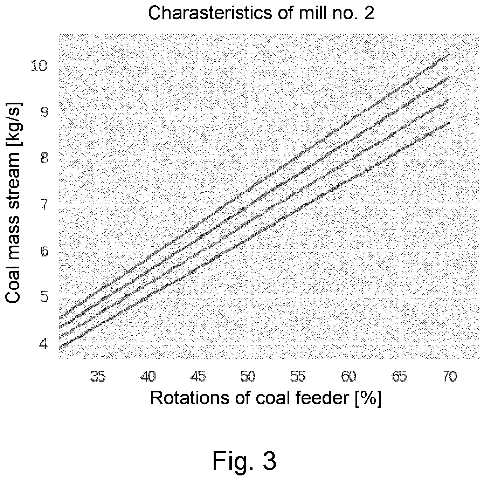

In step 207, for the steady states of boiler operation provided in step 206, linear mill characteristics 1-4 are created as shown in Fig. 3 (determining the dependence of the mass flow [expressed in t/h] on the load of the mill feeder motors (which is translated to coal feeder revolutions) [expressed in %]) based on determination coefficient metric R2.

As a result, in step 208, a set of mill characteristics is obtained, i.e. individual characteristics for different fuel qualities, which are a measure of the coal's susceptibility to milling, as for example shown in Fig. 3.

In step 209, the current mill characteristics are detected (individually for each mill) based on the measured operational parameters of the coal mill, such as mill motor power, pressure of air entering the mill, revolutions of mill feeders.

On the basis of a particular current mill characteristics, the mass flow of fuel fed by a particular mill is determined in step 210. The characteristic is selected depending on at least one currently measurement parameter of the mill (when the measured parameter is changed, another characteristic is selected).

Then, in step 211, the estimated LHV of the fuel is determined based on the energy balances and the determined boiler efficiency and it is reported as the result of the procedure.

$$LHV = \frac{\dot{Q}_{in}}{\dot{m}_{pal}*\eta_{boiler}}$$ wherein $\dot{m}_{pal}$ is the mass flow of fuel

In step 212, the boiler efficiency for the thus estimated LHV is determined according to the PN-EN 12952-15:2004 standard.

The boiler efficiency is determined on an ongoing basis (on-line) for a particular historical moment, e. g. for every minute. After updating the calorific value, the boiler efficiency is calculated again.

The LHV estimated in step 211 and the boiler efficiency estimated in step 212 are provided as input to step 207, allowing a more accurate determination of the set of characteristics.

Thus, steps 207-212 are performed iteratively until the boiler efficiency calculation error (i. e. the difference between successive iterations of steps 207-212) converges to a value below a predetermined threshold, for example of the order of $10^{-3}$. The calculation error is defined as $|\eta_{boiler_{n-1}} - \eta_{boiler_n}| < 10^{-3}$.

The process described in Fig. 2 in steps 207-212 is implemented on historical data of the block operation (i.e. data collected over a certain period of time, for example several months or several years) and on laboratory data of fuel properties. The historical data and results prepared in this way, in particular the estimated LHV, mill characteristics, fuel flow rate and boiler efficiency (after obtaining the error convergence) are used to train and test an artificial intelligence model configured to predict the current calorific value of the fuel. Other operational data of the block can be used as well, such as operational parameters of turbine, including flows, pressures and temperatures for live steam, superheated steam and secondary steam, operational data of the boiler, operational data of at least one mill, composition of exhaust gases, power of the block.

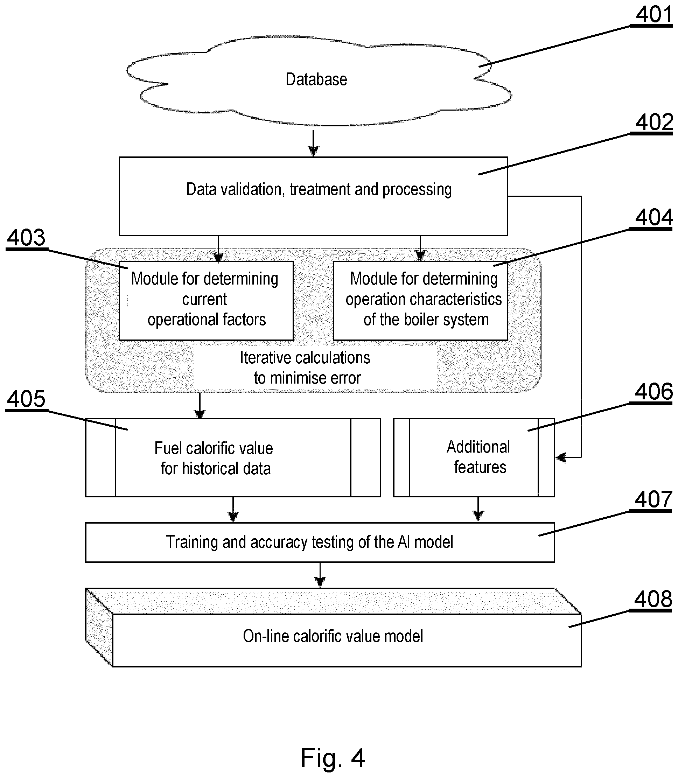

Fig. 4 shows a simplified diagram of the module for on-line determination of calorific value. The data are retrieved from a database 401 (or other type of archive module), both for preparation and on-line operation of the model. In module 402, the data are validated (by removing incorrect indications or by simulating their correct values and processed (e.g. by aggregation or by extracting averages from several measurement sensors) in order to obtain meaningful values for further modules. Based on the processed data in module 403, basic operational factors are determined, such as secondary steam flux (if not measured), boiler thermal power $Q_{in}$, chemical energy of fuel supplied to the boiler $q_{Pal}$, boiler losses and boiler efficiency. In module 404, operation characteristics for the coal mills (as shown in Fig. 3) are determined for steady states of boiler operation, until the error in boiler efficiency calculations is minimised and converged, according to steps 207-212. In module 405, the calorific value is recalculated for historical data which, in combination with the measurement sensor data retrieved in module 406 (i.e.: live and secondary steam parameters, mill system operational parameters, parameters of air supplied to the boiler, flue gas parameters, operating settings, flue gas denitrification system operational parameters, boiler support equipment operational parameters and atmospheric parameters), are used to train the model based on artificial intelligence algorithms 407 to determine the calorific value. The prepared model 408 predicts the current calorific value of the fuel based on the measurement data retrieved in module 406. An artificial intelligence model 408 based on recursive elements is used, which allows the effect of energy accumulation and the response delays of automation and control systems to be included in the model. The module 408 is trained by means of the module 407 such that the historical operational data of the boiler operation with the corresponding calorific value of the fuel is used for training the artificial intelligence model. Then, such trained model 408 can be used to predict on-line (i. e. on an ongoing basis) the calorific value of the fuel based on current operational data.

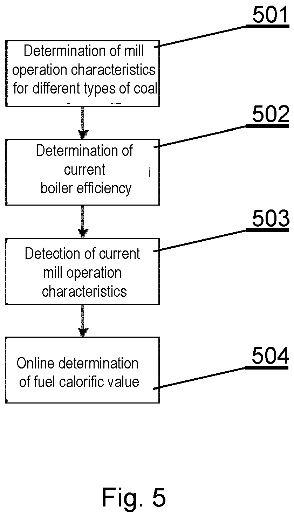

Fig. 5 shows a simplified diagram of the method for determining the calorific value, which is a generalisation of the flowchart in Fig. 2. In step 501, the operation characteristics of the mill 13 for different types of coal are determined. Then, in step 502, current efficiency of the boiler is determined. In step 503, current operation characteristics of the mill 13 are detected, and in step 504, calorific value of the fuel is determined on an ongoing basis (for example, for each minute).

## Claims

1. A method for on-line monitoring and determining a calorific value of a solid fuel that is currently combusted in a boiler that comprises a combustion chamber (10) with a steam production system, to which the solid fuel is fed from at least one mill (13) connected to a hopper (14), **characterised in that** the method comprises the following steps:

- measuring, on-line during the operation of the boiler, operational data of the boiler, and operational data of at least one mill;
- wherein the operational data of the boiler are measured by means of sensors (15-1, 15-2, 15-3, 15-4) and comprise at least one of: temperature, pressure, steam flow; wherein the operational data of the at least one mill are measured by means of sensors (13-1) and comprise at least one of: mill power, air pressure upstream of the mill, solid fuel feeder revolutions, air temperature upstream of the mill, temperature of the dust-air mixture downstream of the mill;
- collecting historical data of measurements of the calorific value of the solid fuel and the operational data of a power block, wherein the operational data of the power block include at least one of: lower heating value (LHV), mill characteristics, fuel flow rate and boiler efficiency, operational parameters of turbine, including flows, pressures and temperatures for live steam, superheated steam and secondary steam, operational data of the boiler, operational data of at least one mill, composition of exhaust gases, power of the block;
- calculating energy balances of the steam production system, based on the collected historical data and depending on the amount of thermal energy input to the turbine generator set (Qin) divided by the boiler efficiency;
- iteratively determining (207-212) an efficiency of the boiler based on the historical data by:

  - determining (208) sets of mill characteristics depending on a calorific value of the solid fuel and operational data of the mill while combusting that solid fuel, wherein the mill characteristics include at least one operational parameter selected from the group consisting of: mill power, air pressure upstream of the mill, solid fuel feeder revolutions, air temperature upstream of the mill, temperature of the dust-air mixture downstream of the mill;
  - determining (210) a fuel mass flux based on the set of mill characteristics (208); and
  - determining (211) the actual calorific value of the fuel (LHV) for the historical data;

- training a model (407) based on artificial intelligence algorithms to predict the calorific value using the historical data of fuel calorific value (211) and measured operational data of the boiler; and
- determining (408) in real time, using the trained model, the calorific value of the solid fuel that is currently combusted.

2. The method according to claim 1, **characterised in that** the historical data of the calorific value of the solid fuel are determined by means of laboratory measurements carried out cyclically or by measurement systems operating continuously, for the solid fuel prior to putting the solid fuel to the hopper (14).

3. The method according to any of previous claims, further comprising measuring, on-line during the operation of the boiler, data of ambient conditions, wherein the data of ambient conditions are measured by means of a sensor (17-1) and comprise at least one of: ambient air temperature, ambient air pressure, ambient air humidity.

4. A system for on-line monitoring and determining a calorific value of a solid fuel that is currently combusted in a boiler that comprises a combustion chamber (10) with a steam production system to which the solid fuel is fed from at least one mill (13) connected to a hopper (14), the system **characterised in that** comprises:

  - an interface to sensors (15-1, 15-2, 15-3, 15-4, 13-1, 17-1) for measuring operational data of the boiler and operational data of at least one mill;

    ◦ wherein the operational data of the boiler comprise at least one of: temperature, pressure, steam flow;
    ◦ wherein the operational data of the at least one mill comprise at least one of: mill power, air pressure upstream of the mill, solid fuel feeder revolutions, air temperature upstream of the mill, temperature of the dust-air mixture downstream of the mill;

  - an archive module (401) for collecting historical data of solid fuel calorific value measurements and operational data of the power block, wherein the operational data of the power block include at least one of: lower heating value (LHV), mill characteristics, fuel flow rate and boiler efficiency, operational parameters of turbine, including flows, pressures and temperatures for live steam, superheated steam and secondary steam, operational data of the boiler, operational data of at least one mill, composition of exhaust gases, power of the block;
  - a module (403) for calculating energy balances of the steam production system on the basis of the historical data collected in the archive module (401) and depending on the amount of thermal energy input to the turbine generator set (Qin) divided by the boiler efficiency;
  - a module (404) for iterative determination (207-212) of boiler efficiency on the basis of historical data, configured to:

- determine (208) sets of mill characteristics depending on a calorific value of the solid fuel and operational data of the mill while combusting that solid fuel, wherein the mill characteristics include at least one operational parameter selected from the group consisting of: mill power, air pressure upstream of the mill, solid fuel feeder revolutions, air temperature upstream of the mill, temperature of the dust-air mixture downstream of the mill;
- determine the fuel mass flux on the basis of the set of mill characteristics (208); and
- determine (211) the actual calorific value of the fuel (LHV) for historical data;

- a model (408) based on artificial intelligence algorithms for predicting calorific value, that is trained (407) using historical data of fuel calorific value (211) and measured operational data of the boiler, and once trained, configured to determine in real time the calorific value of the solid fuel that is currently combusted on the basis of the currently measured operational data of the boiler.

**Patentansprüche**

1. Verfahren zur Online-Überwachung und Bestimmung eines Heizwerts eines Festbrennstoffs, der derzeit in einem Kessel verbrannt wird, der eine Brennkammer (10) mit einem Dampferzeugungssystem umfasst, dem der Festbrennstoff aus mindestens einer Mühle (13) zugeführt wird, die mit einem Trichter (14) verbunden ist, **dadurch gekennzeichnet, dass** das Verfahren die folgenden Schritte umfasst:

- Online-Messen, während des Betriebes des Kessels, von Betriebsdaten des Kessels und von Betriebsdaten von mindestens einer Mühle;
- wobei die Betriebsdaten des Kessels mittels Sensoren (15-1, 15-2, 15-3, 15-4) gemessen werden und mindestens eines umfassen von: Temperatur, Druck, Dampfstrom; wobei die Betriebsdaten der mindestens einen Mühle mittels Sensoren (13-1) gemessen werden und mindestens eines umfassen von: Mühlenleistung, Luftdruck stromaufwärts der Mühle, Drehzahl des Festbrennstoffzuführers, Lufttemperatur stromaufwärts der Mühle, Temperatur des Staub-Luft-Gemisches stromabwärts der Mühle;
- Sammeln historischer Daten von Messungen des Heizwerts des Festbrennstoffs und der Betriebsdaten eines Kraftwerksblocks, wobei die Betriebsdaten des Kraftwerksblocks mindestens eines beinhalten von: unterem Heizwert (LHV), Mühlenkennlinien, Brennstoffdurchflussrate und Kesselwirkungsgrad, Betriebsparameter der Turbine, einschließlich Durchfluss, Druck und Temperatur für Frischdampf, überhitzten Dampf und Sekundärdampf, Betriebsdaten des Kessels, Betriebsdaten von mindestens einer Mühle, Zusammensetzung der Abgase, Leistung des Blocks;
- Berechnen der Energiebilanzen des Dampferzeugungssystems auf Grundlage der gesammelten historischen Daten und abhängig von der Menge der dem Turbinengeneratorsatz zugeführten Wärmeenergie (Qin) geteilt durch den Kesselwirkungsgrad;
- iteratives Bestimmen (207-212) eines Wirkungsgrads des Kessels auf der Grundlage der historischen Daten durch:

- Bestimmen (208) von Sätzen von Mühlenkennlinien in Abhängigkeit von einem Heizwert des Festbrennstoffs und von Betriebsdaten der Mühle während des Verbrennens dieses Festbrennstoffs, wobei die Mühlenkennlinien mindestens einen Betriebsparameter beinhalten, der ausgewählt ist aus der Gruppe bestehend aus: Mühlenleistung, Luftdruck stromaufwärts der Mühle, Drehzahl der Festbrennstoffzuführung, Lufttemperatur stromaufwärts der Mühle, Temperatur des Staub-Luft-Gemisches stromabwärts der Mühle;
- Bestimmen (210) eines Brennstoffmassenstroms auf der Grundlage des Satzes von Mühlenkennlinien (208); und
- Bestimmen (211) des tatsächlichen Heizwerts des Brennstoffs (LHV) für die historischen Daten;
- Trainieren eines Modells (407) auf der Grundlage von Algorithmen künstlicher Intelligenz zur Vorhersage des Heizwerts unter Verwendung der historischen Daten des Heizwerts des Brennstoffs (211) und gemessener Betriebsdaten des Kessels; und
- Bestimmen (408), in Echtzeit unter Verwendung des trainierten Modells, des Heizwerts des aktuell verbrannten Festbrennstoffs.

2. Verfahren nach Anspruch 1, **dadurch gekennzeichnet, dass** die historischen Daten des Heizwerts des Festbrennstoffs mittels zyklisch durchgeführter Labormessungen oder durch kontinuierlich arbeitende Messsysteme für den Festbrennstoff vor dem Einfüllen des Festbrennstoffs in den Trichter (14) bestimmt werden.

3. Verfahren nach einem der vorhergehenden Ansprüche, ferner umfassend das Online-Messen, während des Betriebes des Kessels, von Daten der Umgebungsbedingungen, wobei die Daten der Umgebungsbedingungen mittels eines Sensors (17-1) gemessen werden und mindestens eines umfassen von: Umgebungslufttemperatur, Umgebungsluftdruck, Umgebungsluftfeuchtigkeit.

4. System zur Online-Überwachung und Bestimmung eines Heizwerts eines Festbrennstoffs, der derzeit in einem Kessel verbrannt wird, der eine Brennkammer (10) mit einem Dampferzeugungssystem umfasst, dem der Festbrennstoff aus mindestens einer Mühle (13) zugeführt wird, die mit einem Trichter (14) verbunden ist, wobei das System **dadurch gekennzeichnet ist, dass** es umfasst:

- eine Schnittstelle zu Sensoren (15-1, 15-2, 15-3, 15-4, 13-1, 17-1) zum Messen von Betriebsdaten des Kessels und von Betriebsdaten mindestens einer Mühle;

    o wobei die Betriebsdaten des Kessels mindestens eines umfassen von: Temperatur, Druck, Dampfstrom;
    o wobei die Betriebsdaten der mindestens einen Mühle mindestens eines umfassen von: Mühlenleistung, Luftdruck stromaufwärts der Mühle, Drehzahl der Festbrennstoffzuführung, Lufttemperatur stromaufwärts der Mühle, Temperatur des Staub-Luft-Gemisches stromabwärts der Mühle;

- ein Archivmodul (401) zum Sammeln historischer Daten von Festbrennstoffheizwertmessungen und Betriebsdaten eines Kraftwerksblocks, wobei die Betriebsdaten des Kraftwerksblocks mindestens eines beinhalten von: unterem Heizwert (LHV), Mühlenkennlinien, Brennstoffdurchflussrate und Kesselwirkungsgrad, Betriebsparametern der Turbine, einschließlich Durchfluss, Druck und Temperatur für Frischdampf, überhitzten Dampf und Sekundärdampf, Betriebsdaten des Kessels, Betriebsdaten von mindestens einer Mühle, Zusammensetzung der Abgase, Leistung des Blocks;
- ein Modul (403) zum Berechnen von Energiebilanzen des Dampferzeugungssystems auf der Grundlage der im Archivmodul (401) gesammelten historischen Daten und abhängig von der Menge der dem Turbinengeneratorsatz zugeführten Wärmeenergie (Qin) geteilt durch den Kesselwirkungsgrad;
- ein Modul (404) zur iterativen Bestimmung (207-212) des Kesselwirkungsgrads auf der Grundlage historischer Daten, das konfiguriert ist zum:

    - Bestimmen (208) von Sätzen von Mühlenkennlinien in Abhängigkeit von einem Heizwert des Festbrennstoffs und von Betriebsdaten der Mühle während des Verbrennens dieses Festbrennstoffs, wobei die Mühlenkennlinien mindestens einen Betriebsparameter beinhalten, der ausgewählt ist aus der Gruppe bestehend aus: Mühlenleistung, Luftdruck stromaufwärts der Mühle, Drehzahl der Festbrennstoffzuführung, Lufttemperatur stromaufwärts der Mühle, Temperatur des Staub-Luft-Gemisches stromabwärts der Mühle;
    - Bestimmen des Brennstoffmassenstroms auf der Grundlage des Satzes von Mühlenkennlinien (208); und
    - Bestimmen (211) des tatsächlichen Heizwerts des Brennstoffs (LHV) für historische Daten;
    - ein Modell (408) zur Vorhersage des Heizwerts, das auf Algorithmen künstlicher Intelligenz basiert und das unter Verwendung historischer Daten des Heizwerts des Brennstoffs (211) und gemessener Betriebsdaten des Kessels trainiert (407) wird und nach dem Training dazu konfiguriert ist, den Heizwert des aktuell verbrannten Festbrennstoffs auf der Grundlage der aktuell gemessenen Betriebsdaten des Kessels in Echtzeit zu bestimmen.

**Revendications**

1. Procédé permettant la surveillance en ligne et la détermination d'une valeur calorifique d'un combustible solide en cours de combustion dans une chaudière qui comprend une chambre de combustion (10) avec un système de production de vapeur, aliment en combustible solide à partir d'au moins un broyeur (13) relié à une trémie (14), **caractérisé en ce que** le procédé comprend les étapes suivantes :

    - la mesure, en ligne durant le fonctionnement de la chaudière, des données de fonctionnement de la chaudière et des données de fonctionnement d'au moins un broyeur ;
    - lesdites données de fonctionnement de la chaudière étant mesurées au moyen de capteurs (15-1, 15-2, 15-3, 15-4) et comprenant au moins un parmi : une température, une pression et un flux de vapeur ; lesdites données de fonctionnement du ou des broyeurs étant mesurées au moyen de capteurs (13-1) et comprenant au moins un parmi : une puissance de broyeur, une pression de l'air en amont du broyeur, des rotations de dispositif d'alimentation en combustible solide, la température de l'air en amont du broyeur, la température du mélange poussière-air en aval du broyeur ;

- la collecte de données historiques de mesures de la valeur calorifique du combustible solide et les données de fonctionnement d'un bloc de puissance, lesdites données de fonctionnement du bloc de puissance comprenant au moins un parmi : la valeur calorifique inférieure (LHV), des caractéristiques du broyeur, le débit de combustible et le rendement de la chaudière, des paramètres de fonctionnement de la turbine, comprenant des flux, des pressions et des températures pour la vapeur vive, la vapeur surchauffée et la vapeur secondaire, des données de fonctionnement de la chaudière, des données de fonctionnement d'au moins un broyeur, la composition des gaz d'échappement, la puissance du bloc ;

- le calcul de bilans énergétiques du système de production de vapeur, sur la base des données historiques collectées et en fonction de la quantité d'énergie thermique introduite dans l'ensemble groupe turbogénérateur (Qin) divisée par le rendement de la chaudière ;

- la détermination de manière itérative (207-212) d'un rendement de la chaudière sur la base des données historiques par :

  - la détermination (208) des ensembles de caractéristiques du broyeur en fonction d'une valeur calorifique du combustible solide et des données de fonctionnement du broyeur pendant la combustion de ce combustible solide, lesdites caractéristiques de broyeur comprenant au moins un paramètre de fonctionnement sélectionné dans le groupe constitué de : la puissance de broyeur, la pression de l'air en amont du broyeur, les rotations du dispositif d'alimentation en combustible solide, la température de l'air en amont du broyeur, la température du mélange poussière-air en aval du broyeur ;
  - la détermination (210) d'un flux massique de combustible sur la base de l'ensemble de caractéristiques de broyeur (208) ;
  et
  - la détermination (211) de la valeur calorifique réelle du combustible (LHV) pour les données historiques ;
  - l'entraînement d'un modèle (407) sur la base des algorithmes d'intelligence artificielle pour prédire la valeur calorifique à l'aide des données historiques de la valeur calorifique de combustible (211) et des données de fonctionnement mesurées de la chaudière ; et
  - la détermination (408) en temps réel, à l'aide du modèle entraîné, de la valeur calorifique du combustible solide en cours de combustion.

2. Procédé selon la revendication 1, **caractérisé en ce que** les données historiques de la valeur calorifique du combustible solide sont déterminées au moyen de mesures en laboratoire effectuées de manière cyclique ou par des systèmes de mesure fonctionnant en continu, pour le combustible solide avant de mettre le combustible solide dans la trémie (14).

3. Procédé selon l'une quelconque des revendications précédentes, comprenant en outre la mesure, en ligne durant le fonctionnement de la chaudière, de données de conditions ambiantes, lesdites données de conditions ambiantes étant mesurées au moyen d'un capteur (17-1) et comprenant au moins un parmi : la température de l'air ambiant, la pression de l'air ambiant et l'humidité de l'air ambiant.

4. Système permettant la surveillance en ligne et la détermination de la valeur calorifique d'un combustible solide en cours de combustion dans une chaudière qui comprend une chambre de combustion (10) avec un système de production de vapeur qui est alimenté en combustible solide à partir d'au moins un broyeur (13) relié à une trémie (14), le système étant **caractérisé en ce qu'**il comprend :

  - une interface pour des capteurs (15-1, 15-2, 15-3, 15-4, 13-1, 17-1) afin de mesurer des données de fonctionnement de la chaudière et des données de fonctionnement d'au moins un broyeur ;

    ◦ lesdites données de fonctionnement de la chaudière comprenant au moins une parmi : une température, une pression, un flux de vapeur ;
    ◦ lesdites données de fonctionnement du au moins un broyeur comprenant au moins un parmi : la puissance de broyeur, la pression de l'air en amont du broyeur, des rotations du dispositif d'alimentation en combustible solide, la température de l'air en amont du broyeur et la température du mélange poussière-air en aval du broyeur ;

  - un module d'archives (401) destiné à collecter des données historiques de mesures de valeur calorifique de combustible solide et des données de fonctionnement du bloc de puissance, lesdites données de fonctionnement du bloc de puissance comprenant au moins un parmi : la valeur calorifique inférieure (LHV), des caractéristiques de broyeur, le débit de combustible et le rendement de la chaudière, des paramètres de fonctionnement de la turbine, comprenant des flux, des pressions et des températures pour la vapeur vive, la vapeur surchauffée et la vapeur secondaire, des données de fonctionnement de la chaudière, des données de fonctionnement d'au moins un broyeur, la composition des gaz d'échappement, la puissance du bloc ;

- un module (403) destiné à calculer des bilans énergétiques du système de production de vapeur sur la base des données historiques collectées dans le module d'archives (401) et en fonction de la quantité d'énergie thermique apportée à l'ensemble groupe turbogénérateur (Qin) divisée par le rendement de la chaudière ;

- un module (404) destiné à la détermination itérative (207-212) du rendement de la chaudière sur la base de données historiques, configuré pour :

- déterminer (208) des ensembles de caractéristiques de broyeur en fonction d'une valeur calorifique du combustible solide et des données de fonctionnement du broyeur pendant la combustion de ce combustible solide, lesdites caractéristiques de broyeur comprenant au moins un paramètre de fonctionnement sélectionné dans le groupe constitué de : la puissance de broyeur, la pression de l'air en amont du broyeur, les rotations du dispositif d'alimentation en combustible solide, la température de l'air en amont du broyeur, la température du mélange poussière-air en aval du broyeur ;

- déterminer le flux massique de combustible sur la base de l'ensemble de caractéristiques (208) de broyeur ; et

- déterminer (211) la valeur calorifique réelle du combustible (LHV) pour des données historiques ;

- un modèle (408) basé sur des algorithmes d'intelligence artificielle destiné à prédire la valeur calorifique, qui est entraîné (407) à l'aide de données historiques de la valeur calorifique du combustible (211) et de données de fonctionnement mesurées de la chaudière, et une fois entraîné, configuré pour déterminer en temps réel la valeur calorifique du combustible solide en cours de combustion sur la base des données de fonctionnement actuellement mesurées de la chaudière.

Fig. 1

204

mills have
identical
characteristics

206

203

steady states
- moving window

clearing movement data
of non-operating mills

201

208

determination
of the boiler
useful
thermal power
Qin

202

adjustment of
the sum of
feeder rpm
to the energy
balance
Qin/boiler_eff
based on
LHV_lab

for steady states,
linear mill
characteristics
determined
- checking on $R^2$

a set of curves -
potential characteristics
for different types
of coal are obtained
- 4 states of operation

determination of
boiler efficiency
based on
laboratory data

209

detection of
current
characteristics

205

207

iterations
until the boiler
eff. Changes < $10^{-3}$

210

determination of
fuel mass flux
from each mill

211

determination of
LHV estimated

212

determination of boiler
eff for LHV estimated

Fig. 2

Charasteristics of mill no. 2

Fig. 3

401
Database

402
Data validation, treatment and processing

403
Module for determining current operational factors

404
Module for determining operation characteristics of the boiler system

Iterative calculations to minimise error

405
Fuel calorific value for historical data

406
Additional features

407
Training and accuracy testing of the AI model

408
On-line calorific value model

Fig. 4

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KUREK DAMIAN et al.** Systemy wsparcia decyzji operatora bloku. Zastosowanie metod deterministycznych i nowoczesnych algorytmów AI. *Nowa Energia,* 12 November 2019, (5-6), 19-21